(19) Europäisches Patentamt
European Patent Office
Office européen des brevets

(11) Veröffentlichungsnummer: **0 476 608 A1**

(12) **EUROPÄISCHE PATENTANMELDUNG**

(21) Anmeldenummer: **91115829.3**

(22) Anmeldetag: **18.09.91**

(51) Int. Cl.[5]: **C07C 59/56**, C07C 39/24, C07C 235/34, C07C 69/66, C07C 309/24, C07C 311/13, G03C 7/34

(30) Priorität: **19.09.90 DE 4029709**

(43) Veröffentlichungstag der Anmeldung: **25.03.92 Patentblatt 92/13**

(84) Benannte Vertragsstaaten: **AT BE CH DE DK ES FR GB GR IT LI LU NL SE**

(71) Anmelder: **BOEHRINGER MANNHEIM GMBH**
**Sandhofer Strasse 112-132**
**W-6800 Mannheim-Waldhof(DE)**

(72) Erfinder: **Junius-Comer, Martina, Dr.**
**Am Bodenbach 5**
**W-8127 Iffeldorf(DE)**

Erfinder: **Vogt, Bernd, Dr.**
**Herrestrasse 14**
**W-8132 Tutzing(DE)**
Erfinder: **Herrmann, Rupert, Dr.**
**In der Au 23**
**W-8120 Weilheim(DE)**

(74) Vertreter: **Huber, Bernhard, Dipl.-Chem. et al**
**Patentanwälte H. Weickmann, Dr. K. Fincke F.A. Weickmann, B. Huber Dr. H. Liska, Dr. J. Prechtel Kopernikusstrasse 9 Postfach 86 08 20**
**W-8000 München 86(DE)**

(54) **Substituierte Phenole.**

(57) Die vorliegende Erfindung betrifft ein colorimetrisches Verfahren zum Nachweis einer oxidativen Kupplungsreaktion, worin eine Kupplungskomponente mit einer Entwicklerkomponente in Gegenwart eines Oxidationsmittels zu einem Farbstoff reagiert, und worin man als Kupplungskomponente eine Verbindung der allgemeinen Formel

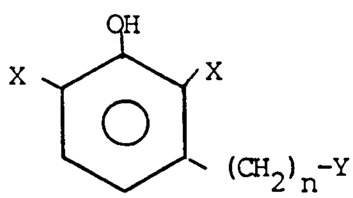

(I')

worin

| | |
|---|---|
| X | Br oder Cl, |
| n | 1 bis 3 ist, |
| Y | $CO_2R$, CONRR', $SO_3R$, $SO_2NRR'$, OR, NRR' oder $NRR'R''^{\oplus}$ ist und |
| R, R', R'' | unabhängig voneinander H oder $C_1$-$C_3$-Alkyl sind, |

verwendet.
Weiterhin ein Gegenstand der vorliegenden Erfindung ist ein Reagenz, welches eine obige Verbindung beinhaltet.

EP 0 476 608 A1

Sowohl für die analytische Chemie als auch für die medizinische Diagnostik ist ein Nachweisverfahren bedeutsam, das als oxidative Kupplung bezeichnet wird. Dabei wird eine Kupplungskomponente mit einer Entwicklerkomponente in Gegenwart eines Oxidationsmittels zu einem Farbstoff umgesetzt. Insbesondere ist der Nachweis von Wasserstoffperoxid mittels geeigneter chromogener Substanzen von hoher Bedeutung. Dies gilt insbesondere für die zahlreichen Nachweisverfahren, bei denen Wasserstoffperoxid als Zwischenprodukt der Reaktion eines Substrats mit der entsprechenden Substratoxidase und Sauerstoff gebildet und im Anschluß in Anwesenheit geeigneter chromogener Reaktionskomponenten, vorwiegend in Gegenwart einer Peroxidase (POD) als Katalysator in eine optisch erfaßbare Verbindung, d.h. in einen Farbstoff überführt wird, wobei die Menge des entstehenden Farbstoffs in quantitativer Beziehung zum gebildeten Wasserstoffperoxid steht. Weiterhin wird Peroxidase häufig als Enzymmarker in Immuntests verwendet und durch Zusatz von Wasserstoffperoxid und von geeigneten chromogenen Reaktionskomponenten nachgewiesen.

Bei der oxidativen Kupplungsreaktion wird eine Kupplungskomponente in Gegenwart einer Entwicklerkomponente zu einem Farbstoff umgesetzt. Für den Nachweis von Wasserstoffperoxid/Peroxidase sind bereits zahlreiche chromogene bzw. Indikator-Systeme genannt und eingesetzt worden. Eines der bekanntesten ist das von Trinder (Ann.Clin.Biochem. 6, 24 (1969) beschriebene Indikator-System, bei dem Phenol als Kupplungskomponente mit 4-Aminoantipyrin als Entwicklerkomponente in Gegenwart von Peroxidase unter Einwirkung von $H_2O_2$ oxidativ zu einem Farbstoff gekuppelt wird. 4-Aminoantipyrin als Entwicklerkomponente kann z.B. auch durch Derivate von Aminoantipyrin, Phenylendiamin oder mit Methylbenzthiazolinhydrazon substituiert werden. Beispiele mit Methylbenzthiazolinhydrazon (MBTH) als Entwickler sind bei N. Gochman et al. (Clin.Chem. 17, 1154 (1971), T.T. Ngo et al., (Anal.Biochem. 105, 389 (1980)) und J. Siedel et al., (Anal.Lett. 21, 1009 (1988)) beschrieben.

Weiterhin kann die oxidative Kupplungsreaktion eingesetzt werden, um bei enzymatischen Reaktionen freigesetzte aromatische Amine zu bestimmen. Für diagnostische Zwecke von besonderer Bedeutung sind der Nachweis von $\gamma$-Glutamyltranspeptidase ($\gamma$-GT) und Leucinaminopeptidase (LAP) sowie der Nachweis von Thrombin. In diesen Fällen wird ein "Peptidamid", dessen Aminosäuresequenz der Spaltstelle des jeweiligen Enzyms entspricht und dessen Amidteil ein aromatisches Amin, insbesondere Phenylendiamin oder Aminophenol oder ein Derivat davon ist, welches als Entwicklerkomponente der oxidativen Kupplung dienen kann, zunächst durch das Enzym gespalten und dann mit einer Kupplungskomponente durch ein Oxidationsmittel zum Farbstoff oxidiert (vgl. DE-A 33 31 588 und EP-A0 076 042).

Die Sensitivität des Nachweises der oxidativen Kupplungsreaktion hängt insbesondere von der verwendeten Kupplungskomponente ab.

Aus der Literatur sind verschiedene Phenolderivate als Kupplungskomponenten für die oxidative Kupplung beschrieben, mit denen empfindliche Nachweissysteme für Wasserstoffperoxid durch Kondensation mit Entwicklerkomponenten wie 4-Aminoantipyrin bzw. substituierten Benzthiazolinonhydrazonen bereitgestellt werden (J.Clin.Chem.Biochem 15 (1977) 699, Anal.Chim. Acta 136 (1982) 121, Anal.Lett. 21, 1009 (1988), EP-A 0 108 526). Die als Kupplungsprodukte entstehenden Farbstoffe weisen einen hohen molaren Extinktionscoeffizienten auf, der sie zum Nachweis der oxidativen Kupplungsreaktion als gut geeignet erscheinen läßt. Nachteilig erwies sich jedoch die hohe Störanfälligkeit dieser Kupplungsprodukte gegenüber Serumbestandteilen, sowie teilweise eine ungenügende Stabilität der Reaktionskomponenten oder der Kupplungsprodukte.

Zum Einsatz als Kupplungskomponente geeignete Phenolderivate werden in der EP-A 0 108 526 beschrieben. Dort offenbarte Phenolderivate, wie z.B. 2,4-Dibrom-3-hydroxybenzoesäure, ergeben jedoch keine reproduzierbaren Ergebnisse. Andere dort offenbarte phenolische Kuppler, wie z.B. 2,4,6-Tribrom-3-hydroxybenzoesäure haben den Nachteil, daß die oxidative Kupplung durch unspezifisch reduzierende Substanzen im Serum oder Urin (z.B. Bilirubin, Ascorbinsäure) und Pharmaka (z.B. $\alpha$-Methyldopa, Ca-Dobesilat) erheblich gestört wird.

Somit besteht ein großes Bedürfnis nach Kupplungskomponenten für die oxidative Kupplungsreaktion, bei denen die Nachteile des Standes der Technik, insbesondere geringe Stabilität und unspezifische Reaktivität mindestens teilweise beseitigt werden.

Die DE- A 23 20 967 offenbart neue Benzylamine mit der allgemeinen Formel

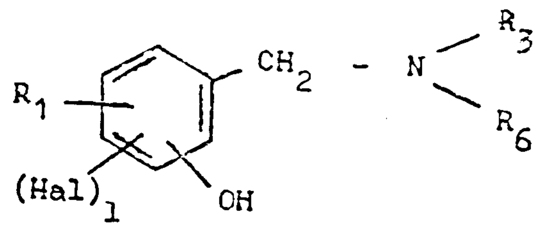

worin $R_1$ ein Wasserstoff-, Chlor- oder Brom-Atom, Hal ein Chlor- oder Bromatom, $R_3$ ein Wasserstoff-Atom, die Methyl- oder Ethylgruppe, $R_6$ den Cyclohexyl-, Hydroxycyclohexyl- oder Morpholinocarbonylmethylrest und 1 die Zahl 1 oder 2 darstellen, und deren physiologisch verträgliche Salze mit anorganischen oder organischen Säuren. Diesen Verbindungen werden pharmakologische Eigenschaften zugeschrieben, neben einer steigernden Wirkung auf die Produktion des Surfaktant oder Antiatelektase-Faktors, insbesondere eine sekretolytische und hustenstillende Wirkung.

Ein Gegenstand der vorliegenden Erfindung ist eine Verbindung der allgemeinen Formel

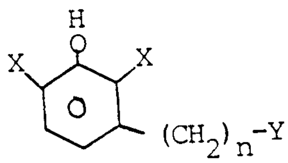

(I)

worin

| | |
|---|---|
| X | Br oder Cl ist, |
| n | 1 bis 3 ist, |
| Y | $CO_2R$, CONRR', $SO_3R$, $SO_2NRR'$ oder OR ist |
| | und |
| R, R' | unabhängig voneinander H oder $C_1$-$C_3$-Alkyl sind. |

In den erfindungsgemäßen 2,6-Dihalogen-3-alkylphenolen wurden überraschenderweise Kupplungskomponenten gefunden, die allen Anforderungen genügen. Sie sind stabil und unempfindlich gegen Autoxidation. Die oxidative Kupplungsreaktion unter Verwendung der erfindungsgemäßen Verbindungen wird nicht durch Störsubstanzen beeinflußt, die in den Körperflüssigkeiten enthalten sein können. Der resultierende Farbstoff ist stabil, besitzt ein günstiges Absorptionsmaximum und einen hohen Extinktionskoeffizienten. Im Test sind Leerwert-Stabilität und Linearität gegeben.

Vorzugsweise bedeutet bei der erfindungsgemäßen Verbindung Y $CO_2R$, besonders bevorzugt $CO_2H$. Vorzugsweise ist n = 1.

Weiterhin beinhaltet die vorliegende Erfindung ein Verfahren zum Herstellen einer Verbindung der allgemeinen Formel

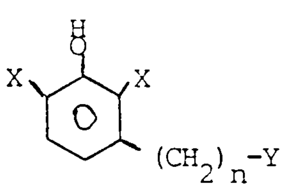

(I)

worin

| | |
|---|---|
| X | Br oder Cl ist, |
| n | 1 bis 3 ist, |
| Y | $CO_2R$, CONRR', $SO_3R$, $SO_2NRR'$ oder OR ist, |
| | und |
| R, R' | unabhängig voneinander H oder $C_1$-$C_3$ Alkyl sind, |

wobei man eine Verbindung

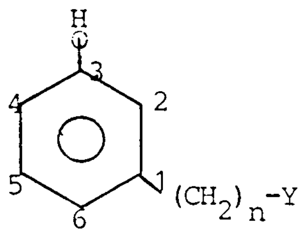

(II)

worin n, Y die oben genannte Bedeutung besitzen, durch Nitrierung an Position 6 des aromatischen Ringes zu einer Verbindung

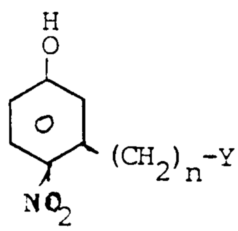

(III)

umsetzt, die Verbindung III durch Bromierung oder Chlorierung an Position 2 und 4 des aromatischen Ringes zu einer Verbindung

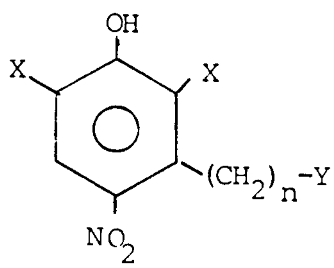

(IV)

umsetzt, worin X Br oder Cl ist und die Nitrogruppe an Position 6 des aromatischen Ringes entfernt, so daß man das Endprodukt (I) erhält.

Der erste Schritt des erfindungsgemäßen Verfahrens ist eine Nitrierung der Ausgangsverbindung II an Position 6 des aromatischen Rings, wobei eine Verbindung III entsteht. Diese Nitrierung kann auf eine dem Fachmann bekannte Weise durch Umsetzen der Ausgangsverbindung mit $HNO_3$, Nitriersäure (ein Gemisch von konzentrierter $HNO_3$ und konzentrierter $H_2SO_4$), Nitroniumtetrafluorborat etc. erhalten werden.

Der zweite Schritt des erfindungsgemäßen Verfahrens ist eine Bromierung oder Chlorierung der Verbindung III an Position 2 und 4 des aromatischen Rings Zu einer Verbindung IV. Die Halogenierung des aromatischen Rings an Position 2 und 4 kann auf eine dem Fachmann bekannte Weise erfolgen. Die Bromierung der Verbindung III erfolgt günstigerweise dadurch, daß man III in wäßriger alkalischer Lösung mit Brom umsetzt. Die Chlorierung erfolgt günstigerweise, indem man III in wäßriger Lösung mit Hypochlorit behandelt.

Der letzte Schritt des Verfahrens zur Herstellung der erfindungsgemäßen Verbindungen ist die Entfernung der Nitrogruppe an Position 6 des aromatischen Ringes. Dies geschieht vorzugsweise so, daß man die Nitrogruppe von IV zu einer Aminogruppe reduziert, diese in eine Diazoniumgruppe überführt und die Diazoniumgruppe reduktiv entfernt. Die Reduktion der Nitrogruppe zu einer Aminogruppe kann z.B. in alkalischer Lösung mit Dithionit oder auch mit Zinn und Salzsäure geschehen. Die Diazotierung des dabei erhaltenen Produkts erfolgt vorzugsweise durch Umsetzen mit Natriumnitrit in Gegenwart von Mineralsäure bei 0 ° C. Die Reduzierung des resultierenden Diazoniumsalzes kann z.B. durch Erhitzen mit hypophosphoriger oder phosphoriger Säure, deren Salzen, einer Natriumstannitlösung oder Ameisensäure erfolgen, wobei man das Diazoniumsalz vorzugsweise mit Natriumhypophosphit erhitzt.

Auf diese Weise sind alle unter die Formel I fallenden Verbindungen erhältlich. Die Herstellung von

Estern bzw. Amiden der 2,4-Dihalogen-3-hydroxyphenyl-carbonsäuren oder -sulfonsäuren erfolgt aus den Säuren durch literaturbekannte und gängige Methoden.

Weiterhin ein Gegenstand der vorliegenden Erfindung ist ein colorimetrisches Verfahren zum Nachweis einer oxidativen Kupplungsreaktion, worin eine Kupplungskomponente mit einer Entwicklerkomponente in Gegenwart eines Oxidationsmittels zu einem Farbstoff reagiert, wobei man als Kupplungskomponente eine Verbindung der allgemeinen Formel

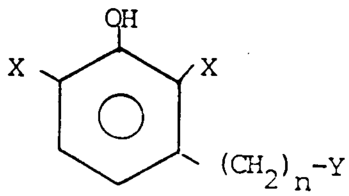

(I')

worin

| | |
|---|---|
| X | Br oder Cl ist, |
| n | 1 bis 3 ist, |
| Y | $CO_2R$, CONRR', $SO_3R$, $SO_2NRR'$, NRR', OR oder $NRR'R''^{\oplus}$ ist und |
| R, R', R'' | unabhängig voneinander H oder $C_1$-$C_3$-Alkyl sind, |

verwendet.

Vorzugsweise verwendet man eine Kupplungskomponente, worin Y $CO_2R$, besonders bevorzugt $CO_2H$ und n = 1 ist. Ist Y = NRR', $NRR'R''^{\oplus}$ erfolgt die Herstellung der Kupplungskomponente vorzugsweise in Analogie zu den in der DE-A 23 20 967 beschriebenen Verfahren.

Bei der oxidativen Kupplungsreaktion ist neben der erfindungsgemäßen Kupplungskomponente eine Entwicklerkomponente erforderlich, die in Gegenwart eines Oxidationsmittels zu einem Farbstoff reagiert. Als Entwicklerkomponenten sind die üblichen Aminoantipyrin-Derivate, Phenylendiamin-Derivate oder Methylbenzthiazolinonhydrazon-Derivate geeignet. Vorzugsweise verwendet man sulfoniertes Methylbenzthiazolinonhydrazon (SMBTH).

Als Oxidationsmittel für die oxidative Kupplungsreaktion verwendet man vorzugsweise das System $H_2O_2$/Peroxid-aktivierendes Mittel. Als Peroxid-aktivierendes Mittel wird vorzugsweise eine Peroxidase verwendet. Andere Peroxid-aktivierende Mittel sind z.B. Wolfram- und Molybdänsäuren und ihre Salze oder ein Gemisch eines Jodids und einer dieser Verbindungen, und Eisen-, Kupfer-, Cerionen und Vanadinsäure. Als Oxidationsmittel kommen weiterhin Peroxide wie Persulfat oder Peracetat sowie Perjodat, Chloramin-T und insbesondere Cyano Ferri-Komplexe, z.B. $K_3Fe(CN)_6$ in Frage. Gemäß DE-A 33 31 588 lassen sich auch Oxidasen einsetzen.

Bei dem erfindungsgemäßen Verfahren zum Nachweis einer oxidativen Kupplung gibt es mehrere prinzipielle Varianten. Man kann entweder

a) die Entwicklerkomponente oder

b) das Oxidationsmittel bzw. eine Komponente des Oxidationsmittels nachweisen. In beiden Fällen wiederum ist sowohl ein direkter als auch ein indirekter Nachweis möglich, d.h. man kann in einem direkten Nachweis die in der Probe vorliegende Konzentration bzw. Menge der Entwicklerkomponente bzw. des Oxidationsmittels bestimmen, während man in einem indireken Nachweis ein System bestimmt, durch welches die Entwicklerkomponente bzw. das Oxidationsmittel in der Probe erzeugt werden.

Somit umfaßt die vorliegende Erfindung in einer ersten bevorzugten Variante ein Verfahren, worin man aromatische Amine, die als Entwicklerkomponenten für die oxidative Kupplung dienen können, oder Systeme, die solche Amine freisetzen, nachweist. Unter Systemen, die aromatische Amine freisetzen, sind insbesondere Substrate zu verstehen, die einer enzymatischen Spaltung zugänglich sind, wodurch dann das als Entwicklerkomponente dienende aromatische Amin gebildet wird.

Vorzugsweise verwendet man als Substrat ein synthetisches Peptidamid. Durch Spaltung eines Peptidamids und der damit verbundenen Freisetzung der Entwicklerkomponente kann man beispielsweise die proteolytischen Enzyme $\gamma$-Glutamyltranspeptidase, Leucinaminopeptidase oder Thrombin nachweisen. Zur Spaltung von Thrombin kann man z.B. als Substrat ein synthetisches Peptidamid Tosyl-Glycyl-Prolyl-Arginin-p-Phenylendiamin oder auch das kommerziell von Boehringer Mannheim erhältliche Chromozym $R^{TH}$ verwenden. Als Oxidationsmittel für diesen Nachweis kann man z.B. $[Fe(CN)_6]^{3-}$, $H_2O_2$/Peroxidase, Perjodat oder Persulfat, vorzugsweise $[Fe(CN)_6]^{3-}$ oder $H_2O_2$/Peroxidase verwenden. Zur Bestimmung anderer Proteasen können entsprechende geeignete synthetische Peptidamide (z.B. die verschiedenen

kommerziell erhältlichen Chromozym-Derivate) eingesetzt werden.

In einer zweiten bevorzugten Variante des erfindungsgemäßen Verfahrens weist man direkt das Oxidationsmittel für die oxidative Kupplungsreaktion oder eine Komponente davon nach. Der Begriff "eine Komponente des Oxidationsmittels" bezieht sich darauf, daß man als Oxidationsmittel Wasserstoffperoxid zusammen mit einer Peroxid-aktivierenden Substanz verwenden kann. Daher kann man sowohl $H_2O_2$ als auch die Peroxid-aktivierende Substanz als Komponenten des Oxidationsmittels nachweisen. In einem direkten Nachweisverfahren bestimmt man vorzugsweise die enzymatische Aktivität von Peroxidase. Dazu wird dem Reaktionsansatz $H_2O_2$, die erfindungsgemäße Kupplungskomponente sowie eine geeignete Entwicklerkomponente zugesetzt. Vorzugsweise weist man eine Peroxidase nach, die an ein anderes Polypeptid, besonders bevorzugt an einen Antikörper oder ein Antikörperfragment gekoppelt ist, so daß die Peroxidaseaktivität mit Hilfe der erfindungsgemäßen Kupplungskomponente zur Quantifizierung von Immuntests bestimmt werden kann.

In einer weiteren bevorzugten Variante des erfindungsgemäßen Verfahrens weist man indirekt ein System nach, welches das Oxidationsmittel bzw. eine Komponente des Oxidationsmittels für die oxidative Kupplung freisetzt. Insbesondere sind hierunter Wasserstoffperoxid produzierende Systeme, z.B. die in der klinischen Diagnostik wichtigen Substrat/Substratoxidase-Paare zu verstehen, bei denen das Substrat in Gegenwart von Luftsauerstoff oxidiert und $H_2O_2$ produziert wird. Es lassen sich somit entweder Substrat oder die Substratoxidasen nachweisen, je nach dem, welche Komponente dem Reagenz zugefügt wird. Als Beispiele für Substrat/Substratoxidasen-Paare sind folgende Wasserstoffperoxid bildende Systeme genannt: Glucose/Glucoseoxidase, Galactose/Galactoseoxidase, L-Aminosäure/L-Aminosäureoxidase, Cholesterin/Cholesterinoxidase, Harnsäure/Urikase, Sarcosin/Sarcosinoxidase, Glycerin/Glycerinoxidase, Glycerinphosphat/Glycerinphosphatoxidase und Pyruvat/Pyruvatoxidase.

Das erfindungsgemäße Nachweisverfahren kann sowohl in der Küvette als auch auf Trockenreagenzträgern durchgeführt werden. Die Quantifizierung der oxidativen Kupplungsreaktion erfolgt dabei mittels Photometern über eine Transmissionsmessung, mit Remissionsphotometern über Remissionsmessung oder mit Hilfe von Vergleichsfarben durch visuellen Vergleich.

Weiterhin ein Gegenstand der vorliegenden Erfindung ist die Verwendung einer Verbindung der allgemeinen Formel I' (s.o.) als Kupplungskomponente zum Nachweis einer oxidativen Kupplungsreaktion, worin die Kupplungskomponente mit einer Entwicklerkomponente in Gegenwart eines Oxidationsmittels zu einem Farbstoff reagiert. Vorzugsweise verwendet man die erfindungsgemäße Kupplungskomponente zum Nachweis von Wasserstoffperoxid oder eines Wasserstoffperoxid freisetzenden Systems, oder zum Nachweis von Peroxidase oder einem anderen Peroxid-aktivierenden Mittel oder zum Nachweis eines aromatischen Amins, das als Entwicklerkomponente geeignet ist, bzw. eines Systems, das ein solches Amin freisetzen kann.

Weiterhin ein Gegenstand der vorliegenden Erfindung ist ein Reagenz zum Nachweis einer oxidativen Kupplungsreaktion, worin eine Kupplungskomponente mit einer Entwicklerkomponente in Gegenwart eines Oxidationsmittels zu einem Farbstoff reagiert, das eine Verbindung der allgemeinen Formel I' als Kupplungskomponente, geeignete Puffer und gegebenenfalls Netzmittel, Aktivatoren oder andere bekannte Hilfsstoffe enthält.

Es lassen sich mit dem erfindungsgemäßen Reagenz Testsysteme herstellen, die in der Küvette vermessen werden. Dazu wird eine erfindungsgemäße Kupplungskomponente zusammen mit den für den jeweiligen Parameternachweis notwendigen Substanzen (Enzyme oder andere Reagenzien), dem Puffer sowie gegebenenfalls Netzmitteln, Aktivatoren und anderen Hilfsstoffen als Pulver vermischt oder zu Tabletten verpreßt oder vorzugsweise in Wasser gelöst und wieder eingetrocknet oder lyophilisiert. Die so gewonnene Reagenzmischung wird vor Gebrauch in Wasser oder einem anderen geeigneten Lösungsmittel gelöst und auf diese Weise die Reagenzlösung bereitgestellt. Nach Vermischen der Probe (Substratlösung, Enzymlösung, Serum oder Plasma) mit einem Aliquot der Reagenzlösung bzw. Reagenzmischung wird die Farbe des durch oxidative Kupplung entstehenden Farbstoffs am Photometer vermessen. Über den molaren Extinktionskoeffizienten und die zugesetzten Reagenz- bzw. Probevolumina wird die jeweilige Konzentration des nachzuweisenden Parameters berechnet. Dabei sind sowohl kinetische als auch Endpunktmessungen möglich.

Ebenso können die erfindungsgemäßen Kupplungskomponenten in Verbindung mit der oxidationsfähigen Entwicklerkomponente zusammen mit Peroxidase, den für den jeweiligen Parameternachweis notwendigen Reagenzien bzw. Enzymen, dem Puffersystem, gegebenenfalls Netzmittels und Aktivatoren sowie anderen Hilfsstoffen auf saugfähigen Reagenzträgern wie Papieren, Vliesen etc. imprägniert werden. Dazu kann man eine oder mehrere Imprägnierlösungen in Form von wäßrigen oder organischen bzw. gemischten Lösungen herstellen, wie es nach der Löslichkeit der Reagenzien oder Hilfsstoffe erforderlich ist. Mit diesen Lösungen werden saug- oder quellfähige Träger, vorzugsweise Filterpapier oder saugfähige Glas- oder

Kunststoffvliesse imprägniert oder besprüht. Anschließend wird getrocknet. Die so hergestellten Reagenzträger können entweder als Schnelldiagnostika zur direkten Bestimmung von Inhaltsstoffen von Flüssigkeiten (z.B. Körperflüssigkeiten wie Blut, Urin oder Speichel oder Lebensmittel wie z.B. Fruchtsäfte, Milch etc.) eingesetzt werden. Dabei wird die zu bestimmende Flüssigkeit mit dem Reagenzträger in Kontakt gebracht. Eine semiquantitative Bestimmung kann durch Vergleich der so entstandenen Farbe mit einer Referenzfarbe geschehen. Eine quantitative Auswertung erfolgt vorzugsweise remissionsphotometrisch. Die remissionsphotometrische Auswertung läßt sich besonders gut durchführen, wenn der Indikator gemeinsam mit den restlichen notwendigen Reagenzien und Hilfsstoffen und einem filmbildenden Kunststoff (z.B. gemäß DE-C 15 98 153) zu einem Reagenzfilm verarbeitet wird.

Für das erfindungsgemäße Reagenz bzw. für das erfindungsgemäße Verfahren geeignete Puffersysteme haben vorzugsweise einen pH-Wert von 6 bis 10, besonders bevorzugt von 6,5 bis 7,5. Am häufigsten werden Phosphat-, Citrat-, Borat- oder GOOD-Puffer mit Alkali- oder Ammoniumgegenionen verwendet. Andere Systeme sind jedoch ebenfalls brauchbar. Netzmittel sind insbesondere anionische und kationische Netzmittel, die mit den erfindungsgemäßen Verbindungen ionische Wechselwirkungen eingehen können. Nichtionische Netzmittel, welche Enzyme aktivieren können, sind jedoch ebenfalls brauchbar. Bevorzugt werden Natriumlaurylsulfat, Dioctylnatriumsulfosuccinat und Alkylarylpolyetheralkohole. Als Aktivatoren sind die für die jeweiligen Substratreaktionen bekannten Aktivatoren einzusetzen. Die oxidative Kupplung verläuft selbst so schnell, daß eine zusätzliche Aktivierung nicht notwendig scheint. Als sonstige Hilfsstoffe können übliche Verdicker, Emulgatoren, optische Aufheller, Kontrastmittel etc. gegebenenfalls eingesetzt werden, wie sie in entsprechenden Tests mit anderen Kupplungskomponenten bekannt sind.

Die Kupplungsreaktion verläuft üblicherweise bei Raumtemperatur, kann jedoch auch ohne weiteres bei höherer Temperatur, z.B. bei 37 ° C, durchgeführt werden, wenn dies für die Reaktionsgeschwindigkeit einer beispielsweise vorgeschalteten enzymatischen Reaktion sinnvoll ist. Für die üblicherweise vorkommenden Reaktionen mit Substraten wie z.B. Enzymen haben sich folgende Konzentrationen der Testlösung bewährt:

| | |
|---|---|
| Kupplungskomponente: | 0,05 bis 100 mmol/l, vorzugsweise 0,1 bis 3 mmol/l |
| Entwicklerkomponente: | 0,05 bis 50 mmol/l, vorzugsweise 0,1 bis 1 mmol/l |
| Puffer: | 0,05 bis 1 mol/l, vorzugsweise 0,1 bis 0,5 mol/l |
| Netzmittel: | 0 bis 1 mol/l, vorzugsweise 0,05 bis 0,1 mol/l |

Wird dem Testreagenz Peroxidase zugesetzt, so erfolgt dies vorzugsweise in einer Konzentration von 1,0 bis 5000 kU/l. Wird dem Reagenz $H_2O_2$ bzw. ein $H_2O_2$-produzierendes Substrat oder eine Substratoxidase zugesetzt, so geschieht dies vorzugsweise in einer Konzentration von 0,1 bis 10 mmol/l. Sonstige Hilfsstoffe können in einer Konzentration von 0 bis 5 mol/l vorhanden sein.

Die genannten Konzentrationsbereiche sind so zu verstehen, daß die unteren Bereiche jeweils für photometrische Tests in der Küvette und die oberen Bereiche für Schnelltests bzw. Tests auf festen Trägern bevorzugt sind.

Für den Nachweis von als Amidasen wirkenden Enzymen wird in der oben genannten Aufstellung die Entwicklerkomponente durch eine entsprechende Menge des Peptidamidsubstrats ersetzt und gegebenenfalls statt $H_2O_2$ eine entsprechende Menge eines anderen Oxidationsmittels, z.B. $K_3FeCN_6$ verwendet.

Die Erfindung soll weiterhin durch die folgenden Beispiele verdeutlicht werden.

Beispiel 1

Herstellung von 2,4-Dibrom-3-hydroxy-phenylessigsäure

a) 3-Hydroxy-6-nitro-phenylessigsäure

15,2 g (100 mmol) 3-Hydroxyphenylessigsäure werden in 15 ml Wasser suspendiert und unter Kühlung langsam 17,5 ml 65 %ige Salpetersäure zugetropft. Anschließend wird 1 Std. bei Raumtemperatur gerührt und auf Eis gegossen. Der Niederschlag wird abfiltriert und mit wenig Essigester gewaschen. Weiteres Produkt ist aus dem Filterat zu gewinnen. Dieses wird auf pH 1 gestellt und mit Chloroform gewaschen, anschließend auf pH 5,5 gestellt und mehrmals mit Essigester ausgeschüttelt. Die vereinigten Essigesterphasen werden mit Wasser gewaschen und aufkonzentriert. Nach Kühlung fällt ein Niederschlag aus, der abfiltriert und mit wenig Essigester gewaschen wird.

| | |
|---|---|
| Ausbeute | 6,8 g (34 %) |
| DC: | $R_f$ = 0,42 (Kieselgel; Chloroform/Methanol 6:1 + 1 % Eisessig) |
| Smp.: | 194 ° C |

b) 2,4-Dibrom-3-hydroxy-6-nitro-phenylessigsäure

10 g (50 mmol) des Produkts aus 1a) werden in 100 ml Wasser und 50 ml 2N Natronlauge gelöst, dann innerhalb von 20 min 6 ml Brom zugetropft. Nach kurzer Zeit fällt ein Niederschlag aus, der abfiltriert und mit Wasser gewaschen wird.

Ausbeute: 16,5 g (92 %)
DC: $R_f$ = 0,4 (Kieselgel; Chloroform/Methanol 6:1 + 1 % Eisessig)

c) 6-Amino-2,4-dibrom-3-hydroxy-phenylessigsäure

16,2 g (46 mmol) des Produkts aus 1b) werden in 140 ml Wasser aufgenommen und mit 277 ml 2N Natronlauge versetzt. Unter Eiskühlung wird portionsweise 57,6 g $Na_2S_2O_4$ zugegeben, dann angesäuert und mehrmals mit Essigester ausgeschüttelt. Die vereinigten Essigesterphasen werden mit Wasser gewaschen und über $Na_2SO_4$ getrocknet. Nach Entfernung des Lösungsmittels bleibt das gewünschte Produkt zurück.

Ausbeute: 8 g (54 %)
DC: $R_f$ = 0,31 (Kieselgel; Chloroform/Methanol 1:1 + 1 % Eisessig)

d) 2,4-Dibrom-3-hydroxy-phenylessigsäure

8 g (24,6 mmol) des Produkts aus 1c) werden in 155 ml 10 % Schwefelsäure aufgenommen und unter Kühlung eine Lösung von 7,5 g $NaNO_2$ in 30 ml Wasser zugetropft. Die entstandene Suspension wird noch 20 min nachgerührt und dann portionsweise zu einer auf 70 °C aufgeheizten Lösung von 38 g Natriumhypophosphit in 15,4 ml Wasser gegeben. Anschließend wird noch 1 Std. bei 70 °C belassen und dann abgekühlt. Der Niederschlag wird abfiltriert und über Kieselgelchromatographie gereinigt (Laufmittel: Essigester/Petrolether 6:2). Das isolierte Produkt wird anschliessend aus Essigester/Hexan umkristallisiert.

Ausbeute: 1,6 g (22 %)
DC $R_f$ = 0,27 (Kieselgel; Essigester/Petrolether 6:1)
Smp.: 186 °C

Beispiel 2

Herstellung von 2,4-Dichlor-3-hydroxy-phenylessigsäure

a) 2,4-Dichlor-3-hydroxy-6-nitro-phenylessigsäure

10,8 g (55 mmol) des Produkts aus 1a) werden in 330 ml Wasser gelöst und mit 180 ml Natriumhypochlorit-Lösung versetzt. Nach einstündigem Rühren wird mit konz. HCl auf pH 1 angesäuert. Der entstandene Niederschlag wird mit HCl und Wasser gewaschen. Aus der Mutterlauge läßt sich durch Ausschütteln mit Essigester noch weiteres Produkt isolieren.

Ausbeute: 10,4 g (71 %)
DC: $R_f$ = 0,4 (Kieselgel; Chloroform/Methanol 6:1 + 1 % Eisessig)

b) 6-Amino-2,4-dichlor-3-hydroxy-phenylessigsäure

Analog Beispiel 1c). Aus 9,5 g (36 mmol) des Produkts aus 2a), 47 g Natriumdithionit, 66 ml Wasser und 137 ml 2N Natronlauge.

Ausbeute: 6,6 g (78 %)
DC: $R_f$ = 0,38 (Kieselgel; Chloroform/Methanol 6:1 + 1 % Eisessig)

c) 2,4-Dichlor-3-hydroxy-phenylessigsäure

Analog Beispiel 1d). Aus 7 g (30 mmol) des Produkts aus 2b) 190 ml 10 % Schwefelsäure, 5,3 g $NaNO_2$, 60 g Natriumhypophosphit. Die Säulenchromatographie wird mit Essigester/Petrolether 1:1 durchgeführt.

Ausbeute:
DC: $R_f$ = 0,44 (Kieselgel; Essigester/Petrolether 1:1)

Beispiel 3

Bestimmung von Creatinin

Testdurchführung:

Enzymatische Creatininbestimmung über Creatininiminohydrolase, N-Methylhydantoinase, N-Carbamoylsarcosinamidohydrolase und Sarcosinoxidase (vergl. J. Siedel et al., Analytical Letters 21, 1009-1017, 1988) am Analysenautomaten Hitachi 704.

350 µl Reagenz 1 (enthaltend 15 U/l Creatininiminohydrolase, 0,5 U/ml N-Methylhydantoinase, 5 U/ml Sarcosinoxidase, 3 U/ml Peroxidase sowie der jeweils untersuchte phenolische Kuppler in 100 mmol/l TRIS HCl pH 7,9) werden zu 7 µl Serumprobe pipettiert. Nach 5-minütiger Vorinkubation wird 70 µl Reagenz 2 (enthaltend 15 U/ml Carbamoylsarcosinamidohydrolase, 0,9 mmol/l MBTH-S (3-Methyl-2-benzo-(2'-sulfo)-thiazolinonhydrazon), 30 mmol/l ATP in 100 mmol/l TRIS HCl pH 7,9) zupipettiert und die Extinktionszunahme bei 546 nm von der 6. bis 10. Minute gemessen. Als phenolischer Farbkuppler wurde jeweils die auf maximale Empfindlichkeit optimierte Menge 2,4-Dibrom-3-hydroxy-phenylessigsäure (2,4 mmol/l) eingesetzt.

Reagenzzusammensetzung:

Reagenz 1

| | |
|---|---|
| 100 mmol/l | TRIS HCl, pH (Raumtemperatur) 7,9 |
| 200 mmol/l | KCl |
| 5 mmol/l | $MgCl_2$ |
| 10 mmol/l | $NH_4Cl$ |
| 10 µmol/l | $K_4Fe(CN)_6$ |
| 15 U/ml | Creatininiminohydrolase |
| 0,5 U/ml | 1-Methylhydantoinase |
| 5 U/ml | Sarcosinoxidase |
| 10 U/ml | Ascorbatoxidase |
| 3 U/ml | Peroxidase |
| 2,4 mmol/l | 2,4-Dibrom-3-hydroxy-phenylessigsäure (2 mmol/l im Test) |

Reagenz 2

| | |
|---|---|
| 100 mmol/l | TRIS HCl, pH (Raumtemperatur) 7,9 |
| 30 mmol/l | ATP |
| 15 U/ml | Carbamoylsarcosinhydrolase |
| 0,9 mmol/l | MBTH-S (0,15 mmol/l im Test) |

Testdurchführung:

Messung am Hitachi 704 Photometer
350 µl Reagenz 1
7 µl Probe
5 min Inkubation
70 µl Reagenz 2
Messung von der 6. bis zur 10. Minute

| | |
|---|---|
| Meßtemperatur: | 37 °C |
| Meßwellenlänge: | 546 nm |
| Referenzwellenlänge: | 700 nm |

Störungen in einem enzymatischen Creatinin-Test

Es wurde eine oxidative Kupplungsreaktion zur Bestimmung von Creatin im Serum durchgeführt. Als Kupplungskomponenten wurden 2,4,6-Tribrom-3-hydroxybenzoesäure (gemäß EP-A0 108 526) und 2,4-Dibrom-3-hydroxy-phenylessigsäure (gemäß Beispiel 1 der vorliegenden Erfindung) verglichen. Als Entwicklerkomponente wurde Methylbenzthiazolinhydrazon-sulfonat eingesetzt.

Das im Test verwendete Serum wurde mit Bilirubin, Hämoglobin, Ascorbinsäure, Ca-Dobesilat und

Methyldopa aufgestockt. Tabelle I zeigt, daß die Creatinin-Wiederfindung bei Verwendung der erfindungsgemäßen Kupplungskommponente in jedem Fall besser als bei Verwendung der bekannten Tribromhydroxbenzoesäure war.

Tabelle I

| Creatinin-Wiederfindung [%] in aufgestockten Humanseren | | |
|---|---|---|
| Störung (Aufstockung) | Tribromhydroxybenzoesäure (gem. EP-A0 108 526) | Dibromhydroxyphenylessigsäure |
| Bilirubin 25 mg/dl | 64,7 | 92,1 |
| Hämolyse 300 mg/dl Hb | 103,8 | 101,8 |
| Ca-Dobesilat 50 mg/l | 95,8 | 96,5 |
| Methyldopa 20 mg/l | 89,7 | 96,3 |
| Ascorbinsäure 30 mg/dl | 97,2 | 100,0 |

Beispiel 4

Herstellung von 2,6-Dibrom-3-hydroxyethyl-phenol

a) 3-Hydroxyethyl-4-nitrophenol

Analog Beispiel 1a) aus 17,5 g 2-(m-Hydroxyphenyl)-ethanol und 42 ml 40 %iger Salpetersäure

Ausbeute: 6,9 g (30 %)
DC: $R_f$ = 0,26 (Kieselgel; Toluol/Essigester 1:4)
Smp.: 167 ° C

b) 2,6-Dibrom-3-hydroxyethyl-4-nitrophenol

Analog Beispiel 1b) aus 10 g des Produkts aus 4a), 110 ml Wasser, 50 ml 2N Natronlauge und 7,2 ml Brom.

Ausbeute: 17,1 9 (93 %)
DC: $R_f$ = 0,1 (Kieselgel; Toluol/Essigester 1:4)
Smp.: 137 ° C

c) 4-Amino-2,6-dibrom-3-hydroxyethyl-phenol

Analog Beispiel 1c) aus 21,8 g des Produkts aus 4b), 200 ml Wasser, 388 ml 2N Natronlauge und 81 g $Na_2S_2O_4$.

Ausbeute: 15,1 g (76 %)
DC: $R_f$ = 0,83 (Kieselgel; Chloroform/Methanol 1:1)

d) 2,6-Dibrom-3-hydroxyethyl-phenol

Analog Beispiel 1d) aus 15,1 g des Produkts aus 4c), 300 ml 10 %iger Schwefelsäure, 7,5 g $NaNO_2$ und 74 g Natriumhypophosphit.

Der Niederschlag wird aus Wasser umkristallisiert.

Ausbeute: 500 mg
DC: $R_f$ = 0,68 (Kieselgel; Toluol/Essigester 1:3)

Beispiel 5

Herstellung von 2,4-Dibrom-3-hydroxyphenyl-propionsäure

5a) 3-Hydroxyphenyl-propionsäure

15 g 3-Hydroxyzimtsäure wird in 300 ml Wasser/Ethanolgemisch 1:1 gelöst und an 0,75 g Palladium/Aktivkohle hydriert, bis kein Verbrauch mehr festzustellen ist. Nach Abfiltrieren des Katalysators wird das Lösungsmittel abgezogen.

Ausbeute: 14,7 g (97 %)

DC: $R_f$ = 0,63 (Kieselgel; Chloroform/Methanol 4:1)

Die folgenden Synthesestufen werden in Analogie zu vorher beschriebenen Beispielen hergestellt. Die Daten sind in unten stehender Tabelle zusammengefaßt.

5b) 3-Hydroxy-6-nitrophenyl-propionsäure (analog Beispiel 1a))
5c) 2,4-Dibrom-3-hydroxy-6-nitrophenyl-propionsäure (analog Beispiel 1b))
5d) 6-Amino-2,4-dibrom-3-hydroxy-phenylpropionsäure (analog Beispiel 1c))
5e) 2,4-Dibrom-3-hydroxyphenylpropionsäure (analog Beispiel 1d))

Tabelle

| Präparat | Ausbeute [%] | DC = $R_f$ [Kieselgel] | Laufmittel |
|---|---|---|---|
| 5b) | 19 | 0,53 | Chloroform/Methanol 7:1 + 1 % Essigsäure |
| 5c) | 50 | 0,15 | Chloroform/Methanol 4:1 |
| 5d) | 49 | 0,23 | Chloroform/Methanol 4:1 |
| 5e) | 10 | 0,28 | Essigester/Petrolether 6:1 |

Beispiel 6

2,4-Dibrom-3-hydroxyphenyl-ethylsulfonsäure

6a) 3-Hydroxyphenyl-ethylensulfonsäure

Die Herstellung erfolgt aus 3-Hydroxybenzaldehyd und Sulfoessigsäure durch Knoevenagel Kondensation analog G. Jones, Org. Reactions 15, 204 (67).

Die folgenden Synthesestufen werden in Analogie zu vorher beschriebenen Beispielen hergestellt.

6b) 3-Hydroxyphenyl-ethylsulfonsäure (analog Beispiel 5a))
6c) 3-Hydroxy-6-nitrophenyl-ethylsulfonsäure (analog Beispiel 1a))
6d) 2,4-Dibrom-3-hydroxy-6-nitrophenyl-ethylsulfonsäure (analog Beispiel 1b))
6e) 6-Amino-2,4-dibrom-3-hydroxyphenyl-ethylsulfonsäure (analog Beispiel 1c))
6f) 2,4-Dibrom-3-hydroxyphenyl-ethylsulfonsäure (analog Beispiel 1d))

**Patentansprüche**

**1.** Verbindung der allgemeinen Formel

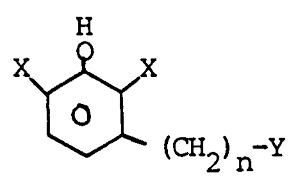

I

worin

X Br oder Cl,
n 1 bis 3 ist,
Y $CO_2R$, CONRR', $SO_3R$, $SO_2NRR'$ oder OR ist und
R, R' unabhängig voneinander H oder $C_1$-$C_3$-Alkyl sind.

**2.** Verbindung nach Anspruch 1,
**dadurch gekennzeichnet**,
daß Y $CO_2R$ ist.

3. Verbindung nach Anspruch 1 oder 2,
**dadurch gekennzeichnet**,
daß n = 1 ist.

4. Verfahren zum Herstellen einer Verbindung der allgemeinen Formel

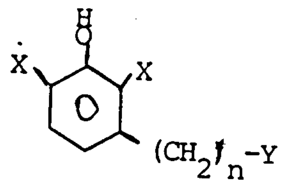

(I)

worin

X Br oder Cl,
n 1 bis 3 ist,
Y $CO_2R$, CONRR', $SO_3R$, $SO_2NRR'$ oder OR ist, und
R, R' unabhängig voneinander H oder $C_1$-$C_3$ Alkyl sind,

**dadurch gekennzeichnet**,
daß man eine Verbindung

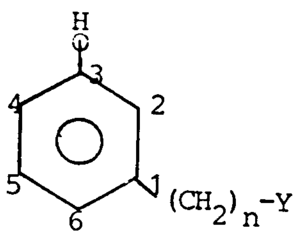

(II)

worin n, Y die oben genannte Bedeutung besitzen, durch Nitrierung an Position 6 des aromatischen Ringes zu einer Verbindung

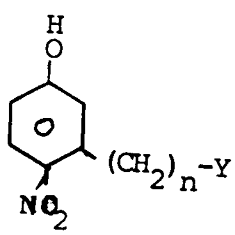

(III)

umsetzt, die Verbindung III durch Bromierung oder Chlorierung an Position 2 und 4 des aromatischen Ringes zu einer Verbindung

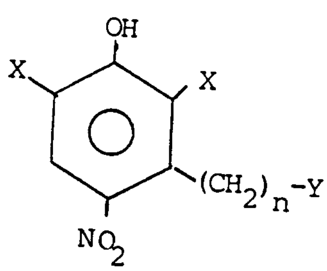

(IV)

umsetzt, worin X Br oder Cl ist und die Nitrogruppe an Position 6 des aromatischen Ringes entfernt, so daß man das Endprodukt (I) erhält.

5. Verfahren nach Anspruch 4,
**dadurch gekennzeichnet**,
daß man die Nitrogruppe von (IV) zu einer Aminogruppe reduziert, diese in eine Diazoniumgruppe überführt und anschließend die Diazoniumgruppe reduktiv entfernt.

6. Colorimetrisches Verfahren zum Nachweis einer oxidativen Kupplungsreaktion, worin eine Kupplungskomponente mit einer Entwicklerkomponente in Gegenwart eines Oxidationsmittels zu einem Farbstoff reagiert,
**dadurch gekennzeichnet**,
daß man als Kupplungskomponente eine Verbindung der allgemeinen Formel

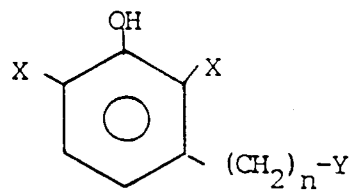

(I')

worin
X Br oder Cl,
n 1 bis 3 ist,
Y $CO_2R$, CONRR', $SO_3R$, $SO_2NRR'$, OR, NRR' oder $NRR'R''^{\oplus}$ ist und
R, R', R'' unabhängig voneinander H oder $C_1$-$C_3$-Alkyl sind,
verwendet.

7. Verwendung einer Verbindung der allgemeinen Formel

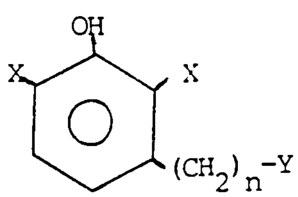

(I')

worin
X Br oder Cl,
n 1 bis 3 ist,
Y $CO_2R$, CONRR', $SO_3R$, $SO_2NRR'$,OR, NRR' oder $NRR'R''^{+}$ ist und
R, R', R'' unabhängig voneinander H oder $C_1$-$C_3$-Alkyl sind,
als Kupplungskomponente zum Nachweis einer oxidativen Kupplungsreaktion, worin die Kupplungskomponente mit einer Entwicklerkomponente in Gegenwart eines Oxidationsmittels zu einem Farbstoff reagiert.

8. Verwendung nach Anspruch 7 ,
**dadurch gekennzeichnet**,
daß man bei der oxidativen Kupplungsreaktion Wasserstoffperoxid oder ein Wasserstoffperoxid freisetzendes System nachweist.

9. Reagenz zum Nachweis einer oxidativen Kupplungsreaktion, worin eine Kupplungskomponente mit einer Entwicklerkomponente in Gegenwart eines Oxidationsmittels zu einem Farbstoff reagiert, enthaltend eine Verbindung der allgemeinen Formel

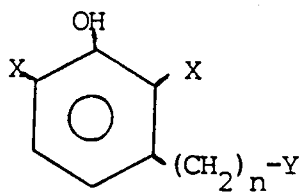

(I')

worin

- X Br oder Cl,
- n 1 bis 3 ist,
- Y $CO_2R$, CONRR', $SO_3R$, $SO_2NRR'$,OR, NRR' oder $NRR'R''^+$ ist und
- R, R', R'' unabhängig voneinander H oder $C_1$-$C_3$-Alkyl sind,

als Kupplungskomponente, geeignete Puffer und gegebenenfalls Netzmittel, Aktivatoren oder andere bekannte Hilfsstoffe.

**10.** Reagenz nach Anspruch 9 ,
**dadurch gekennzeichnet**,
daß es die Kupplungskomponente in einer Konzentration von 0,05 bis 100 mmol/l, vorzugsweise von 0,1 bis 3 mmol/l in der Testlösung enthält.

Europäisches
Patentamt

# EUROPÄISCHER RECHERCHENBERICHT

Nummer der Anmeldung

**EP 91 11 5829**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | DE-A-2 644 194 (KONISHIROKU PHOTO INDUSTRY)<br>* Seite 15, Beispiel 30; Ansprüche 1,2 *<br>– – – | 1,6,7,9 | C 07 C 59/56<br>C 07 C 39/24<br>C 07 C 235/34<br>C 07 C 69/66<br>C 07 C 309/24<br>C 07 C 311/13<br>G 03 C 7/34 |
| A | EP-A-0 175 151 (BAYER AG)<br>* Seiten 8-12; Ansprüche 1,5,10 *<br>– – – | 4,6 | |
| A | EP-A-0 071 569 (CIBA-GEIGY AG)<br>* Seiten 25,26; Anspruch 1 *<br>– – – | 1,4,6,7 | |
| A | CHEMICAL ABSTRACTS Band 84, Nr. 17, 26. April 1976, Seite 502, rechte Spalte, Zusammenfassung Nr. 121383v, Columbus, Ohio, US; K. KROHN: "Synthesis of bacteriostatic 2,4-dibromohomogentisic acid amides and related compounds."<br>& Tetrahedron Lett. 1975, Nr. 52, Seiten 4667-4668<br>– – – | 1 | |
| A | CHEMICAL ABSTRACTS Band 65, Nr. 3, 1. August 1966, Spalte 3783, Zusammenfassung Nr. 3783b, Columbus, Ohio, US; M.S. GIBSON et al.: "A synthesis of Beta-(2,4-dibromo-5-methoxylphenyl)propionic acid using diimide, and some observations on para-benzyloxyphenylacetic acid."<br>& J. Chem. Soc. C. Org. 1966, Nr. 13, Seiten 1206,1207<br>– – – | 1 | **RECHERCHIERTE SACHGEBIETE (Int. Cl.5)** |
| A,D | EP-A-0 108 526 (BECKMAN INSTRUMENTS IND.)<br>* Zusammenfassung *<br>– – – – – | 1,6 | C 07 C 59/00<br>C 07 C 39/00<br>C 07 C 235/00<br>C 07 C 69/00<br>C 07 C 309/00<br>C 07 C 311/00<br>G 03 C 7/00 |

Der vorliegende Recherchenbericht wurde für alle Patentansprüche erstellt

| Recherchenort | Abschlußdatum der Recherche | Prüfer |
|---|---|---|
| Berlin | 14 November 91 | RUFET J.M.A. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument